# EUROPEAN PATENT SPECIFICATION

(11) **EP 0 731 071 B1**
(45) Date of publication and mention of the grant of the patent: **03.01.2001**
(21) Application number: 96301516.9
(22) Date of filing: 06.03.1996
(51) Int. Cl.: C07C 15/08, C07C 6/12

(54) **Process for producing xylene**
Verfahren zur Herstellung von Xylol
Procédé pour la préparation du xylène

(30) Priority: 06.03.1995 JP 7458795
(43) Date of publication of application: 11.09.1996
(73) Proprietor: TORAY INDUSTRIES, INC., Tokyo 103-8666 (JP)
(72) Inventor: Ichioka, Ryoji, Nagoya-shi, Aichi, 457 (JP); Yamakawa, Shinobu, Nagoya-shi, Aichi, 457 (JP); Okino, Hirohito, Otsu-shi, Shiga, 520 (JP)
(74) Representative: Coleiro, Raymond

(56) References cited:
- DE-A- 2 005 820
- DATABASE WPI Section Ch, Week 8109 Derwent Publications Ltd., London, GB; Class E14, AN 81-14634D XP002003107 & JP-A-55 164 631 (TORAY IND INC) , 23 December 1980

## Description

The present invention relates to a process for efficient production of xylene from feedstock containing C₉ alkyl aromatic hydrocarbons (which are generally regarded as useless) by disproportionation, transalkylation (sometimes referred to as "rearrangement"), and dealkylation.

Xylene as a feedstock for p-xylene and o-xylene is usually produced from naphtha by reforming, followed by extraction and fractionation, or by extraction and fractionation of cracked gasoline as a by-product of thermal cracking of naphtha. Xylene is also produced on an industrial scale from toluene or a mixture of toluene and C₉ aromatic hydrocarbons by disproportionation and transalkylation of alkyl groups, such as is described in DE-A- 2 005 820. However, toluene itself is an industrially important raw material for the production of benzene by dealkylation.

On the other hand, JP-B-48413/1974 and JP-B-16782/1975 each disclose a process for producing C₁₀ aromatic hydrocarbons (such as durene) from C₉ aromatic hydrocarbons (including propylbenzene isomers, methylethylbenzene isomers and trimethylbenzene isomers) by disproportionation and transalkylation. However, nothing is known about the efficient production of xylene from feedstock containing one or more C₉ aromatic hydrocarbon(s).

JP-A-55 164 631 discloses the transalkylation of alkylaromatic hydrocarbons using a catalyst containing mordenite and rhenium components.

A known process for industrially producing xylene from toluene and C₉ aromatic hydrocarbons with the aid of amorphous silica-alumina catalyst is disclosed in PETROTECH, (1970), 2(12), 1160. This process suffers the disadvantage that the catalyst has to be continuously regenerated by using a moving bed so as to maintain a certain level of yield and activity.

A process for producing xylene from C₉ aromatic hydrocarbons alone or in combination with toluene with the aid of a zeolite catalyst is reported by I. Wang et al., Ind. Chem. Res., (1990), 29, 2005. This process does not necessarily provide a satisfactory yield.

So far, there has been no efficient process of producing xylene from C₉ aromatic hydrocarbons.

The present invention addresses the problem of providing a process for efficiently producing xylene by disproportionation, transalkylation and dealkylation from a feedstock comprising one or more C₉ aromatic hydrocarbon(s), which are generally regarded as useless.

Thus, we have found that it is possible to produce xylene efficiently from a feedstock comprising at least one C₉ aromatic hydrocarbon by disproportionation, transalkylation, and dealkylation if an aromatic hydrocarbon having one or more ethyl group(s) is present, in the feedstock, in a certain amount.

The present invention provides a process for producing xylene from a feedstock comprising at least one C₉ (alkyl aromatic) hydrocarbon with the aid of a mordenite zeolite catalyst capable of effecting disproportionation, transalkylation and dealkylation, the feedstock containing, in an amount of 5 to 50% by weight of the total weight of the feedstock, an aromatic hydrocarbon having at least one ethyl group.

The feedstock is preferably substantially free from toluene and is also preferably composed mainly of at least one C₉ (alkyl aromatic) hydrocarbon, such that the total amount of C₉ (alkyl aromatic) hydrocarbon represents the component of the feedstock present in the largest (i.e. at least equal largest) amount, more preferably at least 50%, especially at least 65%, by weight of total feedstock.

The feedstock used in a process of the invention contains an aromatic hydrocarbon having one or more ethyl group(s), which is exemplified by ethylbenzene, methylethylbenzene, dimethylethylbenzene and diethylbenzene.

When the feedstock contains methylethylbenzene as aromatic hydrocarbon having an ethyl group, this represents only at least a constituent of the C₉ (alkyl aromatic) component. However, preferably, the feedstock then comprises additionally another C₉ (alkyl aromatic) hydrocarbon, for example trimethylbenzene.

Thus, using a process in accordance with the invention, xylene can be produced efficiently from a feedstock comprising at least one C₉ (alkyl aromatic) hydrocarbon with the aid of a mordenite zeolite catalyst capable of effecting disproportionation, transalkylation and dealkylation, provided that the feedstock contains an aromatic hydrocarbon having one or more ethyl group(s) and present in the feedstock in an amount of 5 to 50 wt%, more preferably 15 to 50 wt%, based on the total weight of the feedstock.

The mordenite zeolite catalyst used in the invention must be capable of effecting disproportionation, transalkylation and dealkylation. It should preferably include a matrix support for the zeolite.

The zeolite contains at least one metal selected the VIB, VIIB and VIII Groups, in an amount of 0.001-5 wt%, preferably 0.02-1 wt% (measured as element content) of the total catalyst. A preferred example of the metal is rhenium, but other suitable metals are, for example, Ni, Co, Mo, Cr and W. The metal may be present in the form of a compound of the metal such as an oxide, nitrate or ammonium compound.

The reaction involving the above-mentioned catalyst is preferably carried out in the presence of hydrogen at 1-6 MPa and 300-550°C and at a WHSV (weight hourly space velocity) of 0.1-10/hr.

Preferred embodiments of the invention will now be described with reference to the following Examples.

### Example 1

A pasty mixture was prepared by mixing from 105 g of powdery synthetic mordenite (sodium form), 45 g of α-alumina, 12 g of alumina sol (containing 10 wt% alumina), 10.5 g of alumina gel (containing 70 wt% alumina), and an adequate amount of deionized water. After kneading for about 2 hours, the pasty mixture was moulded into cylindrical pellets, each measuring 1.0 mm long and 1.2 mm in diameter. The pellets were dried at 120°C for 16 hours. The dried pellets (50 g in an absolute dry condition at 520°C) were baked at 400°C for 5 hours in an atmosphere of air. After cooling, the baked pellets were treated with 100 g of 10 wt% aqueous solution of ammonium chloride at 80-85°C for 1 hour. The treated pellets were strained off the solution and thoroughly washed with water. The pellets were treated with 100 g of 5 wt% aqueous solution of tartaric acid at 80 to 85°C for 3 hours. The treated pellets were strained off the solution and thoroughly washed with water. The washed pellets were dipped in 6.5 g of 5 wt% aqueous solution of rhenium(VII) oxide (Re₂O₇) at room temperature for impregnation with rhenium. The pellets were dried again at 120°C for 16 hours and then baked at 540°C for 8 hours in an atmosphere of air. Thus, a hydrogen ion exchanged mordenite catalyst (A) was obtained. This catalyst (A) contained 0.25 wt% of rhenium (in an absolute dry condition at 520°C).

Using this catalyst (A) in a fixed-bed catalytic reactor, xylene was produced from feedstock composed of trimethylbenzene (TMB) as a C₉ (alkyl aromatic) hydrocarbon and methylethylbenzene (ET) as an aromatic hydrocarbon having an ethyl group in varied ratios. The reaction conditions were as follows:

| | |
|---|---|
| Temperature | 400°C |
| Pressure | 4 MPa |
| WHSV | 2.5 h⁻¹ |
| H2/feedstock | 4.0 mol/mol |

The results are shown in Table 1. It is noted that the yield of xylene increases as the amount of ET increases up to 50 wt%. However, beyond this limit, the yield of xylene decreases.

**Table 1**

| Run No. | Ratio (by weight) of ET/(TMB+ET) in feedstock | Amount (g) of xylene produced per 100 g of feedstock |
|---|---|---|
| 1 (comparative example) | 0 | 20 |
| 2 | 0.25 | 31 |
| 3 | 0.45 | 34 |
| 4 (comparative example) | 0.65 | 28 |

### Example 2

Using the catalyst (A) in a fixed-bed catalytic reactor, xylene was produced in the same manner as in Example 1 from feedstock in which ET was replaced by ethylbenzene (EB) or diethylbenzene (DEB).

The results are shown in Table 2. It is noted that the yield of xylene is favorably affected by both EB and DEB.

**Table 2**

| Run No. | Composition (by weight) of feedstock | Amount (g) of xylene produced per 100 g of feedstock |
|---|---|---|
| 1 | TMB+EB (EB/TMB=30/70) | 34 |
| 2 | TMB+DEB (DEB/TMB=35/65) | 34 |

### Example 3

Catalysts were prepared in the same manner as in Example 1 except that the amount of rhenium was varied. Using the catalysts in a fixed-bed catalytic reactor, xylene was produced in the same manner as in Example 1 from the same feedstock as used in Run No. 3 in Example 1.

The results are shown in Table 3. It is noted that the yield of xylene increases with the increasing amount of rhenium in the range of 0.01 wt% to 0.02 wt%. The effect of rhenium levels off beyond 0.10 wt%.

**Table 3**

| Run No. | Content of rhenium as element (wt%) | Amount (g) of xylene produced per 100 g of feedstock |
|---|---|---|
| 1 (comparative example) | 0 | 20 |
| 2 | 0.01 | 23 |
| 3 | 0.02 | 32 |
| 4 | 0.10 | 34 |
| 5 | 0.20 | 34 |

### Example 4

Six catalysts (B to G) were prepared, each containing rhenium, nickel, cobalt, molybdenum, chromium and tungsten respectively. The first four catalysts (B to E) were prepared in the same manner as in Example 1 by impregnation with an aqueous solution containing the respective metal element. The last two catalysts (F and G) were also prepared in the same manner as in Example 1 except that the compound shown in Table 4 was incorporated into the catalyst components at the time of mixing.

**Table 4**

| Catalyst | Metal | Compound | Incorporated by |
|---|---|---|---|
| B | Re | Re₂O₇ | Dipping and impregnation |
| C | Ni | Ni(NO₃)₂6H₂O | Dipping and impregnation |
| D | Co | Co(NO₃)₂6H₂O | Dipping and impregnation |
| E | Mo | (NH₄)₆Mo₇O4H₂O | Dipping and impregnation |
| F | Cr | CrO₃ | Mixing |
| G | W | WO₃ | Mixing |

Using each catalyst (B to G) in a fixed-bed catalytic reactor, xylene was produced under the same conditions as in Example 1 from the same feedstock as used in Run No. 3 in Example 1. The results are shown in Table 5. It is noted that the catalyst containing rhenium is most active with the minimal content.

**Table 5**

| Catalyst | Metal | Content (wt%) of metal (as element) in catalyst | Amount (g) of xylene produced from 100 g of feedstock |
|---|---|---|---|
| B | Re | 0.15 | 34 |
| C | Ni | 0.40 | 30 |
| D | Co | 0.40 | 26 |
| E | Mo | 0.40 | 32 |
| F | Cr | 0.40 | 28 |
| G | W | 0.24 | 22 |

### Example 5

Three catalysts, each containing a different amount of rhenium, were prepared in the same manner as in Example 1. Using each catalyst in a fixed-bed catalytic reactor, xylene was produced under the same conditions as in Example 1 from the same feedstock as used in Run No. 3 in Example 1. The rate of decrease in yield was recorded. The results are shown in Table 6. It is noted that the catalyst becomes less liable to deterioration in proportion to the amount of rhenium contained therein.

**Table 6**

| Content (wt%) of rhenium (as element) in catalyst | Decrease in yield of xylene (wt% per day) |
|---|---|
| 0 (comparative example) | 1.50 |
| 0.01 | 0.96 |
| 0.20 | less than 0.04 |

## Claims

1. A process for producing xylene from a feedstock comprising at lease one C₉ (alkyl aromatic) hydrocarbon with the aid of a mordenite zeolite catalyst capable of effecting disproportionation, transalkylation and dealkylation, said catalyst including at least one metal selected from the VIB, VIIB and VIII Groups, in an amount (as element content) of 0.001 to 5 wt% of the total weight of total catalyst, wherein an aromatic hydrocarbon having at least one ethyl group is present in the feedstock in an amount of 5 to 50 wt% of the total weight of the feedstock.

2. A process according to Claim 1, wherein the aromatic hydrocarbon having at least one ethyl group is present in the feedstock in an amount of 15 to 50 wt% of the total weight of the feedstock.

3. A process according to Claim 1, wherein the metal is rhenium.

4. A process according to Claim 3, wherein the mordenite zeolite contains rhenium in an amount (as element content) of 0.02 to 1 wt% by weight of the total weight of total catalyst.

5. A process according to any preceding claim which is carried out under a pressure of 1-6 MPa and at a temperature of 300-550°C, in the presence of hydrogen, and at a weight hourly space velocity (WHSV) of 0.1-10/hr.

## Patentansprüche

1. Verfahren zur Herstellung von Xylol aus einem Ausgangsmaterial, das zumindest einen C₉-(Alkyl-Aromat)-Kohlenwasserstoff enthält, mit Hilfe eines Mordenit-Zeolith-Katalysators, der in der Lage ist, Disproportionierung, Umalkylierung und Desalkylierung herbeizuführen, wobei der Katalysator zumindest ein Metall, ausgewählt aus den Gruppen VIB, VIIB und VIII, in einer Menge (berechnet als Elementgehalt) von 0,001 bis 5 Gew.-% des Gesamtgewichts des Gesamt-Katalysators enthält, worin ein aromatischer Kohlenwasserstoff mit zumindest einer Ethylgruppe im Ausgangsmaterial in einer Menge von 5 bis 50 Gew.-% des Gesamtgewichts des Ausgangsmaterials enthalten ist.

2. Verfahren nach Anspruch 1, worin der aromatische Kohlenwasserstoff mit zumindest einer Ethylgruppe im Ausgangsmaterial in einer Menge von 15 bis 50 Gew.-% des Gesamtgewichts des Ausgangsmaterials vorhanden ist.

3. Verfahren nach Anspruch 1, worin das Metall Rhenium ist.

4. Verfahren nach Anspruch 3, worin der Mordenit-Zeolith Rhenium in einer Menge (berechnet als Elementgehalt) von 0,02 bis 1 Gew.-% des Gesamtgewichts des Gesamt-Katalysators enthält.

5. Verfahren nach einem der vorangegangenen Ansprüche, das unter einem Druck von 1 bis 6 MPa und bei einer Temperatur von 300 bis 500 °C in Gegenwart von Wasserstoff und mit einer gewichtsbezogenen stündlichen Raumgeschwindigkeit ("weight hourly space velocity"; WHSV) von 0,1 bis 10 h⁻¹ durchgeführt wird.

## Revendications

1. Procédé pour la production de xylène à partir d'une charge d'alimentation comprenant au moins un hydrocarbure (alkyle aromatique) C₉ avec l'aide d'un catalyseur de zéolithe mordénite capable d'effectuer un disproportionnement, une transalkylation et une désalkylation, ledit catalyseur comprenant au moins un métal sélectionné dans les groupes VIB, VIIB et VIII, en une quantité (en tant que teneur en élément) de 0,001 à 5% en poids du poids total du catalyseur total, où un hydrocarbure aromatique ayant au moins un groupe éthyle est présent dans la charge d'alimentation en une quantité de 5 à 50% en poids du poids total de la charge d'alimentation.

2. Procédé selon la revendication 1, où l'hydrocarbure aromatique ayant au moins un groupe éthyle est présent dans la charge d'alimentation en une quantité de 15 à 50% en poids du poids total de la charge d'alimentation.

3. Procédé selon la revendication 1, où le métal est le rhénium.

4. Procédé selon la revendication 3, où la zéolithe mordénite contient du rhénium en une quantité (en tant que teneur en élément) de 0,02 à 1% en poids du poids total du catalyseur total.

5. Procédé selon toute revendication précédente qui est effectué sous une pression de 1-6 MPa et à une température de 300-550°C, en présence d'hydrogène et à une vitesse spatiale horaire en poids (WHSV) de 0,1-10/h.
